# EUROPEAN PATENT APPLICATION

(11) **EP 4 529 838 A1**
(43) Date of publication of application: **02.04.2025**
(21) Application number: 24202492.5
(22) Date of filing: 25.09.2024
(51) Int. Cl.: A61B 5/145, A61B 5/00, A61B 5/389, A61B 5/369, A61B 5/318, G16H 40/60

(54) **BIOSIGNAL MONITORING SYSTEM WITH DETACHABLE ELECTRONICS AND WIRELESS RECHARGEABLE BATTERY**

(30) Priority: 26.09.2023 US 202363585270 P; 27.06.2024 US 202418757354
(71) Applicant: Renesas Electronics Corporation, Tokyo 135-0061 (JP)
(72) Inventor: NAURATH, Dirk, 40472 Düsseldorf (DE)
(74) Representative: MERH-IP Matias Erny Reichl Hoffmann Patentanwälte PartG mbB

(57) **Abstract**

Systems and methods for sensing and processing biosignals are described. An example system can include a first device configured to sense at least one biosignal and a second device. The second device can receive the at least one biosignal from the first device. The second device can receive power via a first wireless interface. The second device can charge a rechargeable battery using the received power. The second device can receive a signal via the first wireless interface, wherein the signal encodes credentials of a user. The second device can demodulate the signal to decode the user credentials. The second device can authenticate the user credentials. The second device can, in response to authentication of the user credentials, communicate the at least one biosignal to a user device via a second wireless interface.

## Description

### CROSS-REFERENCE TO RELATED APPLICATION

This application claims the benefit under 35 U.S.C. §119(e) of U.S. Provisional Application No. 63/585,270, filed on September 26, 2023. The entire disclosure of U.S. Provisional Application No. 63/585,270 is incorporated by reference herein.

### BACKGROUND

The present disclosure relates in general to systems and devices that implement biosignal monitoring systems, such as continuous glucose monitoring systems, with detachable electronics and wireless rechargeable battery.

Biosignal monitoring systems can include sensors and various electronics to measure biosignals. An example of a biosignal monitoring system can be continuous glucose monitoring (CGM) system for continuously monitoring blood sugar. Measurement and monitoring of biosignals such as glucose level can be vital for users with specific medical conditions such as diabetes. A continuous biosignal monitoring system can provide noninvasive, autonomous and continuous monitoring without a need for users to perform invasive measures such as manually obtaining blood samples from the users. Some continuous biosignal monitors can be implanted in, or placed on, the skin of patients, such as the upper arm, for example, and can penetrate the skin with electrodes. These continuous biosignal monitors may need to be removed periodically, such as every two weeks, and disposed of completely - including the electronics in the biosignal monitors. The disposal of the biosignal monitors including the electronics can incur considerable costs and can increase environmental pollution.

### BRIEF DESCRIPTION OF THE DRAWINGS

Fig. 1 is a diagram showing a conventional biosignal monitoring system.
Fig. 2 is a diagram showing an example of a biosignal monitoring system with detachable electronics and wireless rechargeable battery in one embodiment.
Fig. 3 is a diagram showing an example implementation of a biosignal monitoring system with detachable electronics and wireless rechargeable battery in one embodiment.
Fig. 4 is a diagram showing an example implementation of a biosignal monitoring system with detachable electronics and wireless rechargeable battery using a biosignal sensor, a user device and a peripheral device in one embodiment.
Fig. 5A, is a diagram showing secure credential and/or security key exchange between a biosignal monitor and a user device using nearfield wireless interface when implementing a biosignal monitoring system with detachable electronics and wireless rechargeable battery in one embodiment.
Fig. 5B is a diagram showing secure credential and/or security key exchange between a peripheral device and a user device using nearfield wireless interface when implementing a biosignal monitoring system with detachable electronics and wireless rechargeable battery in one embodiment.
Fig. 5C is a diagram showing encrypted data exchange among a biosignal monitor, a peripheral device and a user device using farfield wireless interface when implementing a biosignal monitoring system with detachable electronics and wireless rechargeable battery in one embodiment.
Fig. 6A is a diagram showing credential and/or security key exchange between a biosignal monitor and a peripheral device using nearfield wireless interface when implementing a biosignal monitoring system with detachable electronics and wireless rechargeable battery in one embodiment.
Fig. 6B is a diagram showing data exchange between a biosignal monitor and a peripheral device using farfield wireless interface when implementing a biosignal monitoring system with detachable electronics and wireless rechargeable battery in one embodiment.
Fig. 7 is a diagram showing another example implementation of a biosignal monitoring system with detachable electronics and wireless rechargeable battery in one embodiment.
Fig. 8A is a diagram showing wireless battery charging using a nearfield wireless interface when implementing a biosignal monitoring system with detachable electronics and wireless rechargeable battery in one embodiment.
Fig. 8B is a diagram showing wireless battery charging using the nearfield wireless interface when implementing a biosignal monitoring system with detachable electronics and wireless rechargeable battery in a peripheral device in one embodiment.
Fig. 8C is a diagram showing wired battery charging when implementing a biosignal monitoring system with detachable electronics and wireless rechargeable battery in a peripheral device in one embodiment.
Fig. 9 illustrates a flow diagram of a process to implement a biosignal monitoring system with detachable electronics and wireless rechargeable battery in one embodiment.

### SUMMARY

In one embodiment, a device for sensing and processing biosignals is generally described. The device can include an analog front end configured to receive at least one biosignal from one or more biosignal sensors. The device can further include a wireless communication receiver comprising a first wireless interface. The wireless communication receiver can be configured to receive power via the first wireless interface. The wireless communication receiver can be further configured to charge a rechargeable battery using the received power. The wireless communication receiver can be further configured to receive a signal via the first wireless interface, wherein the signal encodes credentials of a user. The device can further include a second wireless interface. The device can further include one or more controllers configured to demodulate the signal to decode the user credentials. The one or more controllers can be further configured to authenticate the user credentials. The one or more controllers can be further configured to, in response to authentication of the user credentials, communicate the at least one biosignal from the analog front end to a user device via the second wireless interface.

In one embodiment, a system for sensing and processing biosignals is generally described. The system can include a first device configured to sense at least one biosignal. The system can further include a second device configured to receive the at least one biosignal from the first device. The second device can be further configured to receive power via a first wireless interface. The second device can be further configured to charge a rechargeable battery using the received power. The second device can be further configured to receive a signal via the first wireless interface, wherein the signal encodes credentials of a user. The second device can be further configured to demodulate the signal to decode the user credentials. The second device can be further configured to authenticate the user credentials. The second device can be further configured to, in response to authentication of the user credentials, communicate the at least one biosignal to a user device via a second wireless interface.

In one embodiment, a method for operating a biosignal monitoring system is generally described. The method can include receiving at least one biosignal from a biosignal sensor. The method can further include receiving power via a first wireless interface. The method can further include charging a rechargeable battery using the received power. The method can further include receiving a signal via the first wireless interface, wherein the signal encodes credentials of a user. The method can further include demodulating the signal to decode the user credentials. The method can further include authenticating the user credentials. The method can further include, in response to authentication of the user credentials, communicating the at least one biosignal to a user device via a second wireless interface.

Further features as well as the structure and operation of various embodiments are described in detail below with reference to the accompanying drawings. In the drawings, like reference numbers indicate identical or functionally similar elements.

### DETAILED DESCRIPTION

In the following description, numerous specific details are set forth, such as particular structures, components, materials, dimensions, processing steps and techniques, in order to provide an understanding of the various embodiments of the present application. However, it will be appreciated by one of ordinary skill in the art that the various embodiments of the present application may be practiced without these specific details. In other instances, well-known structures or processing steps have not been described in detail in order to avoid obscuring the present application.

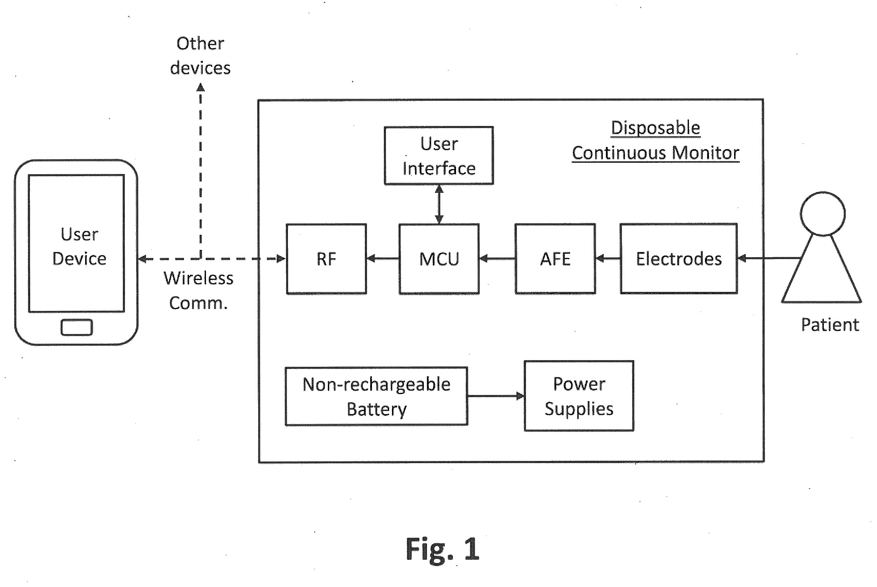

Fig. 1 is a diagram showing a conventional biosignal monitoring system. The conventional biosignal monitoring system in Fig. 1 can be a CGM system including electrodes that can be attached to the skin of a patient, an analog front end (AFE), a microcontroller (MCU), an optional user interface including components such as light emitting diodes (LEDs) and buttons, a non-rechargeable battery, power supply rails and communication interface such as radio frequency (RF) communication interface for communicating with a user device such as mobile phone or insulin pump. The conventional CGM system in Fig. 1 may not be reused, and will be disposed, after a lapse of specific amount of time, such as when sensing electrodes are encapsulated by human immune reaction and/or the non-rechargeable battery is fully discharged.

As shown by the conventional CGM system in Fig. 1, in addition to high cost, every new CGM needs to be paired to an end user application, typically via a smart phone or portable device (e.g., user device shown in Fig. 1). The pairing can be achieved using various authentication schemes, such as a personal identification number (PIN) code. The need to periodically pair new CGM sensors to user devices can become cumbersome and can increase cybersecurity risk, such as increasing the risk of the user device being compromised by nearby Bluetooth sniffers or other hacking. To be described in more detail below, the systems and devices described herein can provide improvement in waste reduction, decrease in cost, increases user convenience, and enhances authentication, security and privacy.

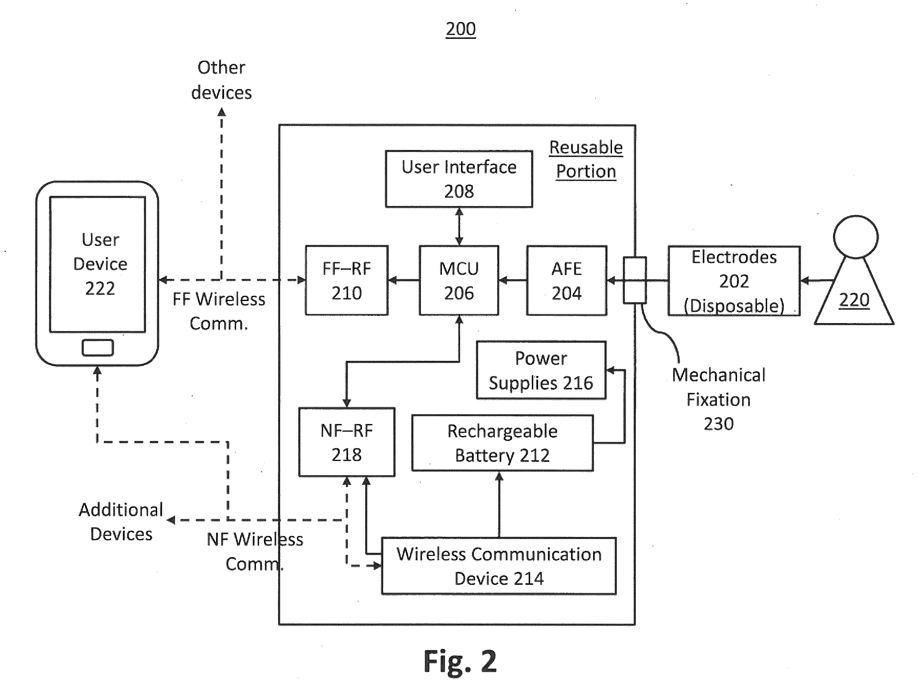

Fig. 2 shows a system block diagram of a biosignal monitoring system with detachable electronics and wireless rechargeable battery in one embodiment. A biosignal monitoring system 200 ("system 200") shown in Fig. 2 can be configured as a CGM system including detachable electronics and wirelessly rechargeable battery. The biosignal monitoring system 200 can also be configured to monitor various types of biosignals, such as glucose values, electrocardiogram (ECG) signals, electroencephalography (EEG) signals, electromyography (EMG) signals, heart rate, oxygen saturation (SpO2), or other biosignals. System 200 can include a reusable portion and a disposable portion. The disposable portion can include a biosignal sensor including one or more electrodes 202 that can be attached to (e.g., implanted in) the skin of a user or patient 220 to obtain biosignals (e.g., glucose values). The reusable portion can include electronic components such as an AFE 204, an MCU or controller 206, a user interface 208 optionally including light emitting diodes (LEDs) and/or buttons and/or capacitive touch, a rechargeable battery 212, a wireless communication device 214, power supplies 216, a first wireless communication interface 218 ("NF-RF 218") and a second wireless communication interface 210 ("FF-RF 210"). First wireless communication interface 218 can be a nearfield (NF) wireless communication interface such as NFC or Qi. Second wireless communication interface 210 can be a farfield (FF) wireless communication interface such as Bluetooth or WiFi or Long-Term Evolution (LTE), e.g., low power LTE-M or NB-IoT, communication interface. First wireless communication interface 218 can be used for facilitating authentication, exchanging user credentials and/or encryption/decryption keys, and/or communicating low speed data with a user device such as mobile phone or an insulin pump. Electrodes 202 can be detachable from AFE 204 and rechargeable battery 212 can be recharged by wireless communication device 214 via first wireless communication interface 218. In one embodiment, wireless communication device 214 can include a wireless power receiver configured to receive power from a wireless power transmitter. Wireless communication device 214 can use the power received wirelessly to charge rechargeable battery 212. Due to electrodes 202 being detachable from AFE 204, and due to the inclusion of rechargeable battery 212 and wireless communication device 214 in system 200, AFE 204, MCU 206, user interface 208 rechargeable battery 212, wireless communication device 214, power supplies 216 and first wireless communication interface 218 do not need to be disposed and can be reused multiple times. Electrodes 202 can be detached and disposed, and new set of electrodes 202 can be attached to various electronics including AFE 204. Further, the utilization of wireless communication device 214 and first wireless communication interface 218 can allow system 200 to perform communication with user devices, such as a user device 222, to exchange and/or obtain confidential information from the user devices with enhanced security. By way of example, wireless communication device 214 can be a wireless power transmitter that operates under near field communication (NFC^{®}-WLC) protocol or Qi^{®} protocol, which utilizes immanently short range communication links, such that a risk of cybersecurity attack such as man-in-the-middle attacks and Bluetooth sniffing can be reduced.

In one embodiment, electrodes 202 can be a part of a first device, where the first device can be a biosignal sensor that can be disposable. The reusable portions of system 200, such as at least AFE 204, MCU 206, user interface 208 rechargeable battery 212, wireless communication device 214, power supplies 216 and first wireless communication interface 218, can form a second device that is reusable. The first device with disposable parts (e.g., electrodes 202) and the second device with the reusable parts can be detachably attached to each other using at least one mechanical fixation 230. Electrodes 202 can be attached to, and detached from reusable electronic parts of system 200 such as AFE 204 using mechanical fixation 230. Electrical contacts of the first device and the second device are brought into contact upon using the mechanical fixation 230 to attach the first device and the second device. The electrical contacts can be used for determining and/or measuring biosignals, such as glucose values, obtained by electrodes 202 via the AFE 204. By way of example, if user 220 needs to changes electrodes 202, electrodes 202 can be detached from the reusable electronic parts of system 200 such as AFE 204, electrodes 202 can be disposed, and a new set of electrodes, that can be identical to electrodes 202, can be attached to the reusable electronic parts of system 200 such as AFE 204.

System 200 can provide a cost reduction of biosignal monitoring systems since electronics such as AFE 204, MCU 206, user interface 208 rechargeable battery 212, wireless communication device 214, power supplies 216 and first wireless communication interface 218 can be reused, while, in one or more embodiments, electrodes 202 may be the sole component that needs to be disposed and replaced. Further, system 200 can provide positive environmental impact since the electronics and batteries does not need to be disposed and can be reused for a significant amount of time. Furthermore, the reusable electronics can also be sealed as a package, without the electrodes, to improve the reusability (e.g., waterproof and dustproof packaging) of the reusable portion. Still further, the option to wirelessly charge rechargeable battery 212 can provide improved electrical safety over wired charging. In one embodiment, system 200 can be implemented in a peripheral device such as an insulin pump. When system 200 is being implemented in peripheral devices, in addition to wireless charging, rechargeable battery 212 that can be in the peripheral device may also be charged using a wired connection. Example wired connections that can be used for the wired charging can include, for example, various types of universal serial bus (USB) protocols. In embodiments where wired charging is implemented in peripheral devices, the peripheral devices can include power interfaces such as a USB interface or socket.

The utilization of wireless communication device 214 to receive user credentials and/or keys can allow MCU 206 to authenticate the user, get credentials and/or encryption keys before biosignals, such as glucose values, obtained from electrodes 202 are forwarded to the user devices using far-field wireless communication via second wireless communication interface 210, thus reducing cybersecurity risk by using a second physical media. Additionally, wireless communication device 214 can increase the security and ease of use of system 200. Since the electronics in CGM system 200 are reusable, wireless pairing with user device 222 such as a smartphone and/or peripherals devices can be maintained and may not have to be paired again each time electrode 202 is replaced.

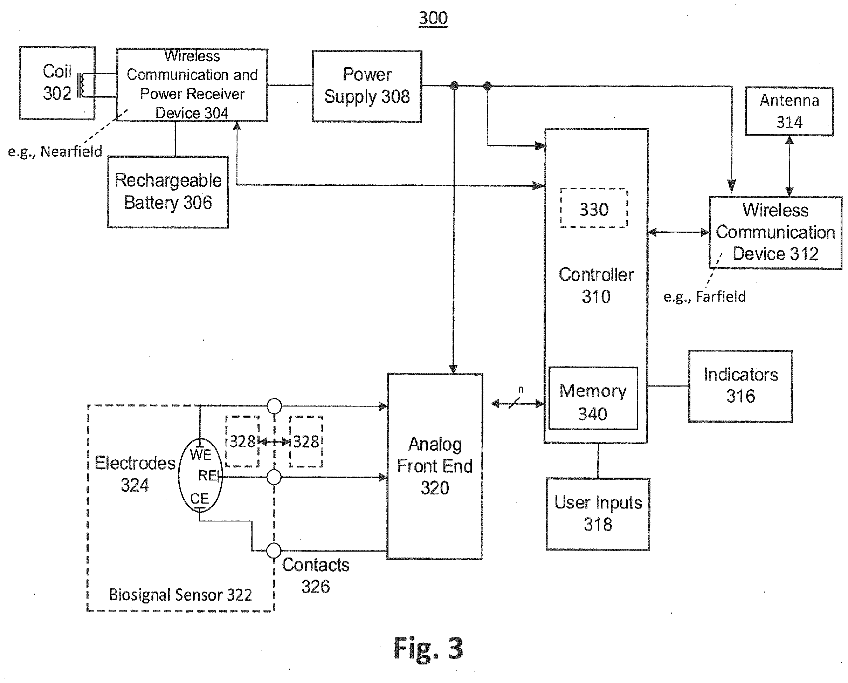

Fig. 3 is a diagram showing an example implementation of a biosignal monitoring system 300 with detachable electronics and wireless rechargeable battery in one embodiment. Descriptions of Fig. 3 can reference components shown in Fig. 2. The implementation of system 300 in Fig. 3 can be an example implementation of system 200 shown in Fig. 2. System 300 shown in Fig. 3 can be a biosignal monitoring system including at least a controller 310, a memory 340, an AFE 320, a coil 302, a wireless communication and wireless power receiver device 304, a rechargeable battery 306, a power supply 308, a wireless communication device 312, an antenna 314, one or more indicators 316, user inputs 318, and a biosignal sensor 322. Controller 310 can be a microcontroller. Coil 302 and wireless communication and wireless power receiver device 304 can be parts of wireless communication device 214 shown in Fig. 2. Wireless communication and wireless power receiver device 304 can be configured to operate and communicate using nearfield wireless communication protocols such as NFC or Qi. Wireless communication device 312 can be configured to operate and communicate using farfield wireless communication protocols such as Bluetooth or WiFi or LTE (e.g., low power LTE-M or NB-IoT). In one embodiment, wireless communication interface 312, antenna 314 and controller 310 can be separated components. Memory 340 can be configured to store a set of instructions including source code and/or executable code, and controller 310 can run the set of instructions stored in memory 340 to execute algorithms and/or applications that carry out the processes, methods, calculations, operations and/or functions described herein. In another embodiment, wireless communication device 312 and/or antenna 314 can be integrated within controller 310. In one embodiment, coil 302, first nearfield wireless communication and wireless power receiver device 304 and rechargeable battery 306 can be external to controller 310 as shown in Fig. 3. In another embodiment, coil 302, wireless communication and wireless power receiver device 304 and rechargeable battery 306 can be integrated with controller 310 as a battery module 330. Rechargeable battery 306 can be, for example, a lithium ion battery having a relatively small size such as button size.

Wireless communication and wireless power receiver device 304 can include a wireless power receiver that include a first wireless interface (e.g., nearfield), and can operate under different wireless power transfer protocols, such as near field communication (NFC), Qi, or other wireless power transfer protocols. In embodiments where wireless communication and wireless power receiver device 304 is external to controller 310, wireless communication and wireless power receiver device 304 can include its own controller. In embodiments where wireless communication and wireless power receiver device 304 is part of controller 310, controller 310 can be configured to operate wireless communication and wireless power receiver device 304. By way of example, when a coil of a wireless power transmitter is within a specific distance from coil 302, the coil of the wireless power transmitter and coil 302 forms a transformer that facilitates power transfer from the wireless power transmitter to wireless communication and wireless power receiver device 304. The limited communication range of nearfield communication protocol can decrease the risk of security breach or potential hacking attacks. Further, analog signals such as amplitude shift keying (ASK) signals can be transmitted from the wireless power transmitter to wireless communication and wireless power receiver device 304 via coil 302 and vice versa. A battery charger in wireless communication and wireless power receiver device 304 can use the wirelessly receive power to charge rechargeable battery 306.

Power supply 308 can provide power to various components in system 300. In one embodiment, power supply 308 can include voltage regulators configure to step up or step down voltages for different components in system 300, such as a DC-DC buck-boost regulator. The voltage regulator can also ensure that all components in system 300 are supplied with a constant voltage as long as rechargeable battery 306 has sufficient charge. Indicators 316 can include, for example, LEDs of different colors that can notify a user about different types of status of system 300. By way of example, indicators 316 can include a LED that can be turned on or flash when rechargeable battery 306 needs to be recharged. In another example, indicators 316 can include another LED that can be turned on when biosignal sensor 322 needs to be detached and replaced. In another example, indicators can include an LED that turns on with a first color to indicate a need to recharge rechargeable battery 306 and turns on with a second color to indicate a need to detach and replace biosignal sensor 322. In another example, the LED can indicate a success or failure of data communications and/or power transfers among wireless communication and wireless power receiver device 304, rechargeable battery 306, and/or external battery charger or transmitter such as wireless charging and nearfield communication device 214 shown in Fig. 2.

User inputs 318 can include switches and/or buttons and/or capacitive touch elements to control different components and operations of system 300. By way of example, user inputs 318 can include a button to turn on or turn off system 300. When system 300 is turned off, power supply 308 and/or rechargeable battery 212 can be disconnected from controller 310 and AFE 320 to ensure a user can safely detach biosignal sensor 322 without the risk of faulty measurements. User inputs 318 can include various other mechanical inputs to alter and/or to be informed about optional display elements of system 300.

Wireless communication device 312 can include a second wireless interface (e.g., farfield) different from the first wireless interface of wireless communication and wireless power receiver device 304. Wireless communication device 312 can operate under a different wireless protocol and/or air interface from wireless communication and wireless power receiver device 304, such as Bluetooth or WiFi or LTE (e.g., low power LTE-M or NB-IoT). By way of example, wireless communication device 312 can be a wireless transceiver that operates under wireless protocols such as Bluetooth or WiFi or LTE (e.g., low power LTE-M or NB-IoT). Wireless communication device 312 can be configured to communicate with user devices outside of system 300 via an antenna 314. Wireless communication device 312 and/or antenna can be separated from controller 310 or can be parts of controller 310. Furthermore, wireless communication device 312 may also have its own integrated microcontroller and/or memory to handle the wireless protocol and tasks offloaded from the main controller 310.

In one embodiment, biosignal sensor 322 can be a glucose monitor including a plurality of electrodes 324. Electrodes 324 can include a working electrode (WE), a reference electrode (RE) and a counter electrode (CE). Electrodes 324 can be attached to (e.g., implanted in) the skin of a patient to obtain glucose values. In one embodiment, biosignal sensor 322 can be detachable from AFE 320 and the rest of the electronics of system 300. In one embodiment, one or more mechanical fixations 328 can be used for connecting biosignal sensor 322 to the electronic components of system 300 including AFE 320. In one embodiment, mechanical fixations 328 can be, for example, magnets such as neodymium magnets. In one embodiment, mechanical fixations 328 can be separable and stable plastic catch with a lock or release lever. Electrodes 324 can be electrically connected to AFE 320 via a plurality of contacts 326. In one embodiment, contacts 326 can be conductive contacts such as gold-plated contacts. Biosignal sensor 322 can be detached from AFE 320.When biosignal sensor 322 is detached, electrodes 324 can also be detached and mechanical fixations 328 on biosignal sensor 322 and AFE 320 can be separated as well.

Due to biosignal sensor 322 being detachable, and due to the inclusion of rechargeable battery 306 and wireless communication and wireless power receiver device 304 for nearfield communication and power transfer, electronic components of system 300 does not need to be disposed and can be reused. Biosignal sensor 322 can be detached and disposed, and a new biosignal sensor 322 can be attached to AFE 320. In one embodiment, AFE 320 can be configured to detect whether biosignal sensor 322 is attached to, or detached from, AFE 320. If biosignal sensor 322 is attached to AFE 320, AFE 320 can receive biosignals measured by electrodes 324 via contacts 326. AFE 320 can measure, evaluate, and/or communicate the received biosignals to controller 310. In one embodiment, the reusable components of system 300 can be sealed as a package such that the reusable components can be waterproof and dustproof.

In one embodiment, when coil 302 is in proximity (e.g., within range of wireless power transfer standards such as NFC or Qi standards) with a user device that includes a transmission coil, the magnetic field between the transmission coil and coil 302 can allow the user device to transmit signals to wireless communication and wireless power receiver device 304 and vice versa. Wireless communication and wireless power receiver device 304 can receive or transmit, from / to a user device, user credentials and/or keys including but not limited to authentication data such as username, password, personal identification number (PIN), encryption keys for obtaining permission or authentication to allow wireless communication device 312 to communicate with the user device. Wireless communication and wireless power receiver device 304 can communicate the user credentials and/or key and/or authentication data to controller 310. Controller 310 can authenticate the user credentials using various authentical methods. In one embodiment, the user device can send a signal encoding the user credentials (e.g., amplitude shift keying (ASK) modulation) and controller 310 can be configured to decode the signal to obtain the user credentials for authentication.

If controller 310 fails to authenticate the user credentials, or the user credentials are incorrect, then controller 310 may not communicate the biosignals from AFE 320 to the user device. If controller 310 authenticates the user credentials, or the user credentials are correct, then controller 310 can pair system 300 with the user device to transfer data via the wireless communication device 312. Controller 310 can communicate the biosignals from AFE 320 to the paired user devices and/or the peripheral devices via wireless communication device 312 and antenna 314. In one embodiment, controller 310 can store the biosignals from AFE 320 in a memory 340 of controller 310 regardless of whether the user credentials are authenticated or not. Some examples of user devices that can be paired with system 300 can include, but not limited to, smartphones, insulin pumps, tablets, laptops, wearable devices, personal computers, or other user devices. The utilization of wireless communication and wireless power receiver device 304 to obtain user credentials can provide energy transfer for charging rechargeable battery 306 and provide secured coupling between user devices and system 300 for communicating sensitive information such as biosignal readings.

Controller 310 can be configured to control various aspects of system 300, such as by running instructions stored in memory 340. In one embodiment, controller 310 can be configured to run applications that controls AFE 320 to obtain biosignals from biosignal sensor 322. Further, controller 310 can be configured to store user credentials and/or encryption keys or alike that are already authenticated such that user devices can remain paired even when biosignal sensor 322 is detached and replaced by a new biosignal sensor.

Advantageously, the utilization of wireless communication and wireless power receiver device 304 in system 300 can enable secured transmission of sensitive information such as user credentials. By way of example, wireless communication and wireless power receiver device 304 can be a near field communication (NFC) or a under the Qi protocol and an appropriate physical interface, such that a risk of cybersecurity attack such as man-in-the-middle attacks and Bluetooth sniffing can be reduced. System 300 can provide other advantages similar to those described for system 200 in Fig. 2.

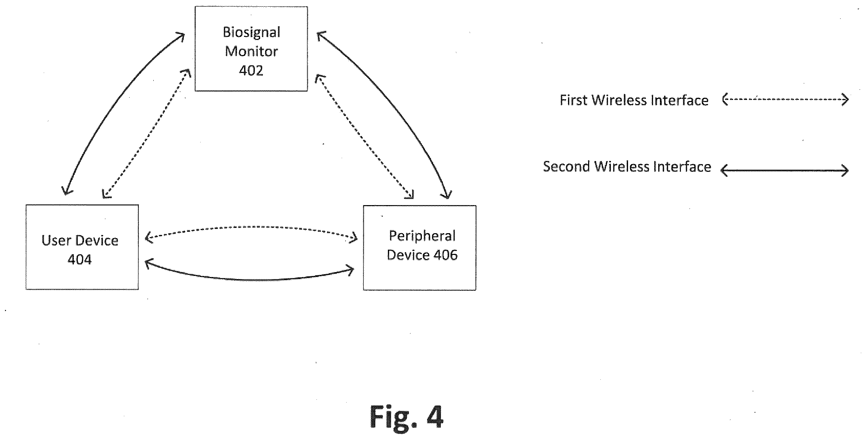

Fig. 4 is a diagram showing an example implementation of a biosignal monitoring system with detachable electronics and wireless rechargeable battery using a biosignal sensor, a user device and a peripheral device in one embodiment. System 200 and 300 shown in Fig. 2 and Fig. 3, respectively, can be implemented as a biosignal monitor 402. The different wireless interfaces utilized in biosignal monitor 402 (or in systems 200, 300) can communicate with a user device 404 (e.g., a smartphone) and a peripheral device (e.g., insulin pump) in one embodiment. In the example embodiment shown in Fig. 4, biosignal monitor 402 can use a first wireless interface that operates within nearfield range, such as NFC or Qi, to communicate with user device 404 and peripheral device 406. Biosignal monitor 402 can also use a second wireless interface that operates within farfield range, such as Bluetooth or WiFi or LTE (e.g., low power LTE-M or NB-IoT), to communicate secured and/or encrypted data with user device 404 and peripheral device 406. User device 404 can also use the first and second wireless interfaces to communicate with peripheral device 406. The capability of biosignal monitor 402 to use both the first and second wireless interfaces can strengthen communication and security among biosignal monitor 402, user device 404 and peripheral device 406. For example, the first wireless interface being an NFC interface can allow biosignal monitor 402 to perform handshaking with user device 404 and peripheral device 406 prior to communicating sensitive information, such as biosignals, from biosignal monitor 402 to user device 404 and peripheral device 406. Also, visualization data and/or control information may be exchanged between the user device 404, the biosignal monitor 402 and/or the peripheral 406.

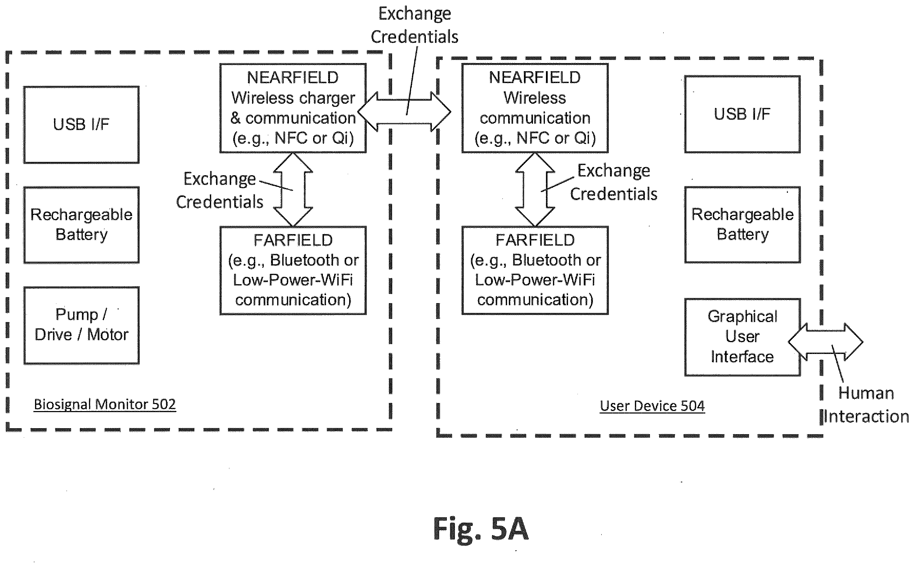

Fig. 5A, is a diagram showing secure credential and/or security key exchange between a biosignal monitor and a user device using nearfield wireless interface when implementing a biosignal monitoring system with detachable electronics and wireless rechargeable battery in one embodiment. In an example embodiment shown in Fig. 5A, a biosignal monitor 502 can implement systems 200, 300 shown in Fig. 2 and Fig. 3, respectively. Biosignal monitor 502 can exchange credentials with a user device 504 that can be a smartphone or alike with graphical user interface (GUI). In one embodiment, biosignal monitor 502 and user device 504 can exchange credentials and/or encryption keys using nearfield wireless communication interface such as NFC or Qi. The exchanged credentials can be authenticated internally in biosignal monitor 502 and user device 504. If both biosignal monitor 502 and user device 504 authenticate the exchanged credentials, then biosignal monitor 502 and user device 504 can be paired. In response to biosignal monitor 502 and user device 504 being paired, biosignal monitor 502 and user device 504 can establish a data communication link and exchange data using farfield wireless communication interfaces such as Bluetooth or WiFi or LTE (e.g., low power LTE-M or NB-IoT). In one embodiment, data communication using the farfield wireless communication interfaces can be secured and/or encrypted using encryption keys that are obtained via the nearfield communication interface. The utilization of two physical wireless communication devices under two different wireless communication protocols, one being nearfield for low speed data (e.g., credentials and/or security keys) and one being farfield for high speed data (e.g., digital data representing biosignals), can decreases the risk of cyber attacks since sensitive information is being exchanged using within nearfield range under a relatively short timeframe and on a different media or wireless channel. In one embodiment, in order to pair biosignal monitor 502 and user device 504, the nearfield wireless communication protocol being used by biosignal monitor 502 and user device 504 needs to be the same. For example, if biosignal monitor 502 is using NFC, user device 504 needs to use NFC to perform the pairing. Further, the pairing occurs prior to any data communications link being established using the farfield wireless communication interface.

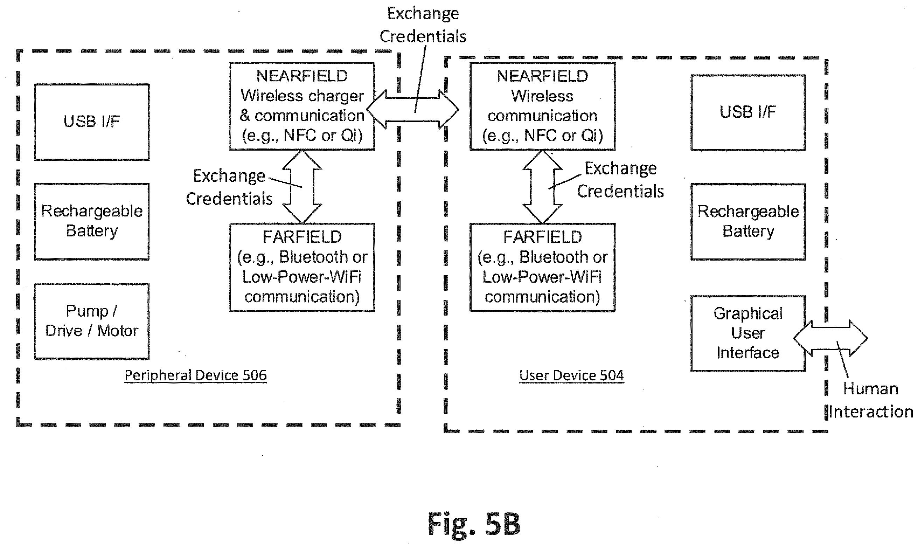

Fig. 5B is a diagram showing secure credential and/or security key exchange between a peripheral device and a user device using nearfield wireless interface when implementing a biosignal monitoring system with detachable electronics and wireless rechargeable battery in one embodiment. A peripheral device 506, such as an insulin pump, can also exchange credentials with user device 504 that can be a smartphone or alike with GUI. In one embodiment, peripheral device 506 and user device 504 can exchange credentials using nearfield wireless communication interface such as NFC or Qi. The exchanged credentials can be authenticated internally in peripheral device 506 and user device 504. If both peripheral device 506 and user device 504 authenticate the exchanged credentials, then peripheral device 506 and user device 504 can be paired. In response to peripheral device 506 and user device 504 being paired, peripheral device 506 and user device 504 can establish a data communication link and exchange secured and/or encrypted data using farfield wireless communication interfaces such as Bluetooth or WiFi or LTE (e.g., low power LTE-M or NB-IoT). In one embodiment, data communication using the established communication link can be secured and/or encrypted using encryption keys that are obtained via the nearfield communication interface. In one embodiment, in order to pair peripheral device 506 and user device 504, the nearfield wireless communication protocol being used by peripheral device 506 and user device 504 needs to be the same. For example, if peripheral device 506 is using NFC, user device 504 needs to use NFC to perform the pairing. Further, the pairing occurs prior to any data communications link being established using the farfield wireless communication interface.

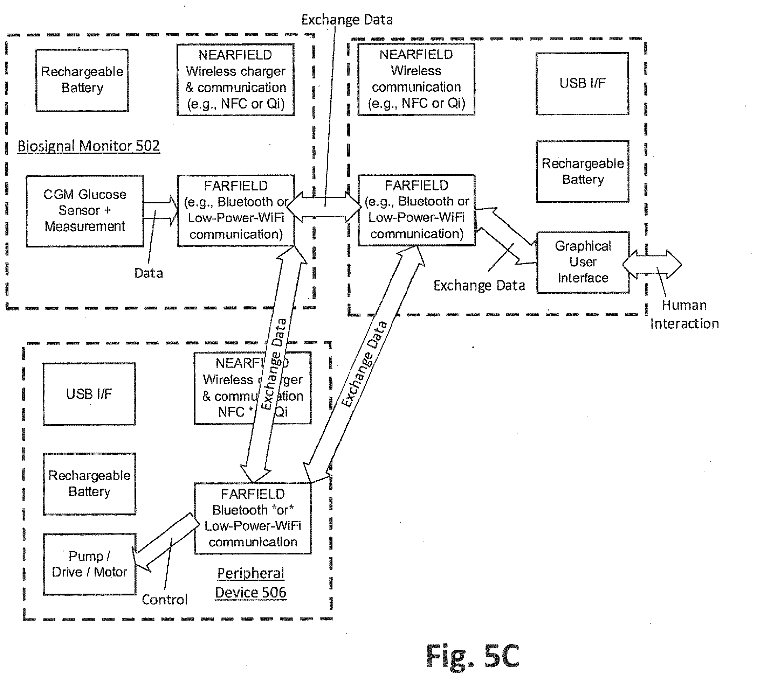

Fig. 5C is a diagram showing encrypted data exchange among a biosignal monitor, a peripheral device and a user device using farfield wireless interface when implementing a biosignal monitoring system with detachable electronics and wireless rechargeable battery in one embodiment. In one embodiment, in response to biosignal monitor 502 and user device 504 being paired, and peripheral device 506 and user device 504 being paired, biosignal monitor 502 can also be paired with peripheral device 506. Secure data communication links using farfield wireless communication interfaces can be established among biosignal monitor 502, user device 504, peripheral device 506. In one embodiment, data communication using the established communication link can be secured and/or encrypted using encryption keys that are obtained via the nearfield communication interface. The established communication link can be used for communicating data, such as biosignals sensed by biosignal monitor 502, from biosignal monitor 502 to user device 504 and to peripheral device 506. In one embodiment, the biosignal signals being provided to peripheral device 506 can provide parameters that may be needed to operate peripheral device 506. By way of example, if peripheral device 506 is an insulin pump, then the biosignals provided to peripheral device 506 can control an amount of insulin to be pumped by peripheral device 506. This injection may need to be approved by the patient using the user device 504 (e.g., according to medical regulation).

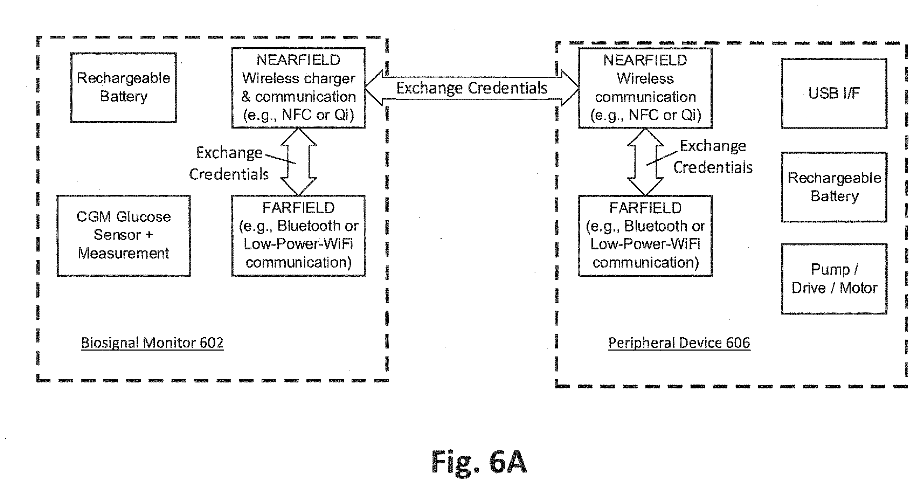

Fig. 6A is a diagram showing credential and/or security key exchange between a biosignal monitor and a peripheral device using nearfield wireless interface when implementing a biosignal monitoring system with detachable electronics and wireless rechargeable battery in one embodiment. In an example embodiment shown in Fig. 6A, a biosignal monitor 602 can implement systems 200, 300 shown in Fig. 2 and Fig. 3, respectively. Biosignal monitor 602 can exchange credentials with a peripheral device 604 that can be an insulin pump. In one embodiment, biosignal monitor 602 and peripheral device 604 can exchange credentials and/or keys using nearfield wireless communication interface such as NFC or Qi. The exchanged credentials can be authenticated internally in biosignal monitor 602 and peripheral device 604. If both biosignal monitor 602 and peripheral device 604 authenticate the exchanged credentials, then biosignal monitor 602 and peripheral device 604 can be paired. In response to biosignal monitor 602 and peripheral device 604 being paired, biosignal monitor 602 and peripheral device 604 can establish a data communication link and exchange data using farfield wireless communication interfaces such as Bluetooth or WiFi or LTE (e.g., low power LTE-M or NB-IoT). In one embodiment, in order to pair biosignal monitor 602 and peripheral device 604, the nearfield wireless communication protocol being used by biosignal monitor 602 and peripheral device 604 needs to be the same. For example, if biosignal monitor 602 is using NFC as the nearfield wireless communication technology, peripheral device 604 needs to use NFC to perform the pairing. Further, the pairing occurs prior to any data communications link being established using the farfield wireless communication interface.

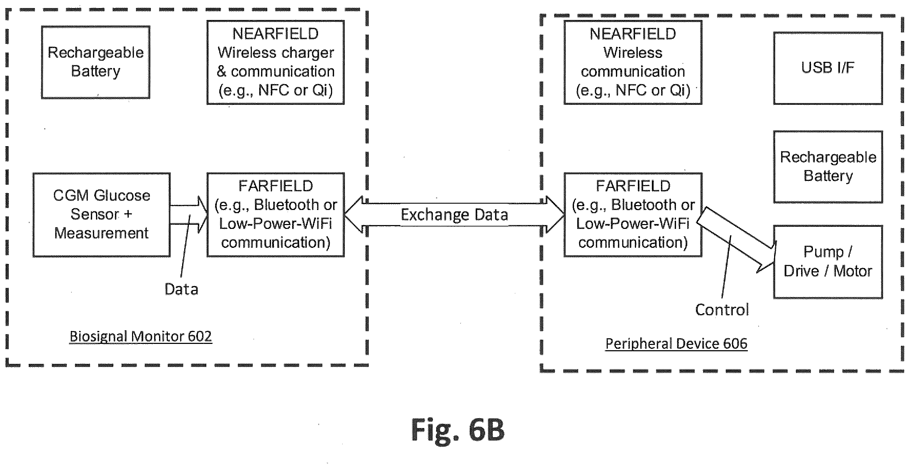

Fig. 6B is a diagram showing data exchange between a biosignal monitor and a peripheral device using farfield wireless interface when implementing a biosignal monitoring system with detachable electronics and wireless rechargeable battery in one embodiment. In one embodiment, in response to biosignal monitor 602 and peripheral device 604 being paired, secure (e.g., secured, encrypted and/or authenticated) data communication link using farfield wireless communication interface can be established among biosignal monitor 602 and peripheral device 604. The established communication link can be used for communicating data, such as biosignals sensed by biosignal monitor 602, from biosignal monitor 602 to peripheral device 604. In one embodiment, the biosignal signals being provided to peripheral device 604 can provide parameters that may be needed to operate peripheral device 604. By way of example, if peripheral device 604 is an insulin pump, then the biosignals provided to peripheral device 604 can control an amount of insulin to be pumped by peripheral device 604.

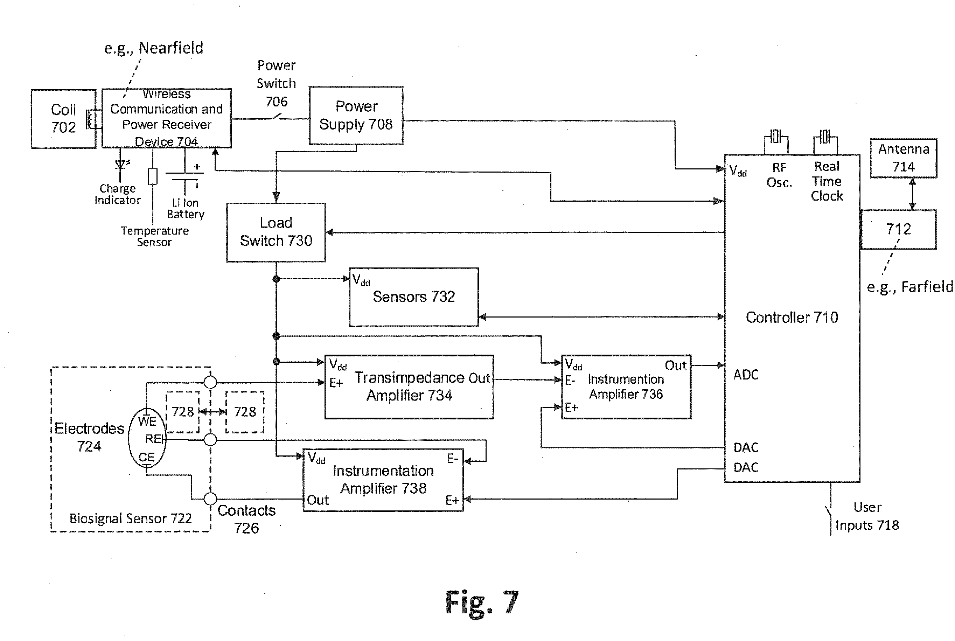

Fig. 7 is a diagram showing another example implementation of a biosignal monitoring system with detachable electronics and wireless rechargeable battery in one embodiment. The implementation of system 700 in Fig. 7 can be applicable to system 200 shown in Fig. 2 and system 300 shown in Fig. 3. System 700 shown in Fig. 7 can be a biosignal monitoring system including at least a controller 710, a coil 702, a wireless communication device and wireless power receiver device 704, a rechargeable battery, a power supply 708, a wireless communication device 712, an antenna 714, user inputs 718, a biosignal sensor 722, a power switch 706, a load switch 730, one or more sensors 732 (e.g. temperature, humidity), a transimpedance amplifier 734, an instrumentation amplifier 736 and an instrumentation amplifier 738. In one embodiment, sensors 732, transimpedance amplifier 734, instrumentation amplifier 736 and instrumentation amplifier 738 can form an AFE, such as AFE 204 and AFE 320 shown in Fig. 2 and Fig. 3, respectively.

Controller 710 can operate in a similar manner as MCU 206 and controller 310 shown in Fig. 2 and Fig. 3, respectively. Coil 702 can be similar to coil 302 shown in Fig. 3. Wireless communication and wireless power receiver device 704 can operate in a similar manner as wireless communication and wireless power receiver device 304 shown in Fig. 3. The rechargeable battery labeled as Li ion in Fig. 7 can be similar to rechargeable battery 212 and 306 shown in Fig. 2 and Fig. 3, respectively. Power supply 708 can operate in a similar manner as power supply 308 shown in Fig. 3. Wireless communication device 712 can operate in a similar manner as wireless communication device 210 and wireless communication device 312 shown in Fig. 2 and Fig. 3, respectively. In one embodiment, Wireless communication device 712 can also be part of controller 710. Antenna 714 can be similar to antenna 314 shown in Fig. 3. User inputs 718 can be similar to user inputs 318 shown in Fig. 3. Biosignal sensor 722 can be similar to biosignal sensor 322 shown in Fig. 3.

Transimpedance amplifier 734 can be an amplifier configured to convert an input current into an output voltage. Instrumentation amplifiers 736, 738 can be precision differential voltage amplifiers that may not be susceptible to common voltages on its inputs. Sensors 732 can include temperature and/or humidity sensors, which may be needed for improved calculation of the biosignals measured by biosignal sensor 722 signal and/or other medical evaluation.

Controller 710 can be configured to control power switch 706, such as by running instructions stored in memory 340 as shown in Fig. 3, to connect or disconnect wireless communication and wireless power receiver device 704. Controller 710 can be further configured to control load switch 730 to connect or disconnect the AFE including sensors 732, transimpedance amplifier 734, instrumentation amplifier 736 and instrumentation amplifier 738. In one embodiment, controller 710 can turn off or open load switch 730 in response to a failure to authenticate user credentials received by wireless communication and wireless power receiver device 704. Controller 710 can turn on or close load switch 730 in response to authenticating user credentials received by wireless communication and wireless power receiver device 704.

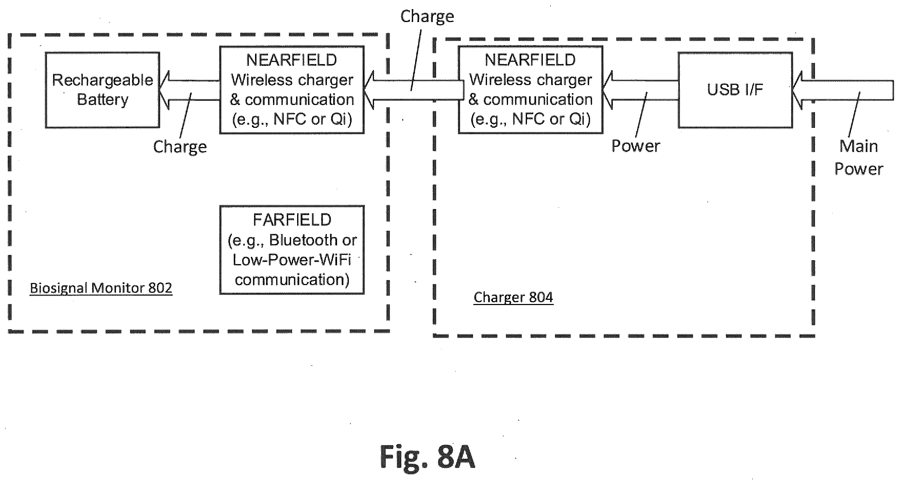

Fig. 8A is a diagram showing wireless battery charging using a first nearfield wireless interface when implementing a biosignal monitoring system with detachable electronics and wireless rechargeable battery in one embodiment. In an example embodiment shown in Fig. 8A, a biosignal monitor 802 can implement systems 200, 300 shown in Fig. 2 and Fig. 3, respectively. Biosignal, monitor 802 can include a rechargeable battery, a nearfield wireless communication interface and a farfield wireless communication interface. To charge the rechargeable battery in biosignal monitor 802, a charger 804 can receive main power from a power source, such as an external battery or a power supply. Charger 804 can include a wireless power transmitter and a nearfield wireless communication interface. The nearfield wireless communication interface in biosignal monitor 802 and charger 804 are the same and can operate under nearfield wireless communication protocols, such as NFC or Qi. When charger 804 is brought into a specific near distance, such as distance that complies with NFC or Qi protocols, charger 804 can wirelessly provide power to charge rechargeable battery in biosignal monitor 802. In one embodiment, while biosignal signal monitor 802 is being charged by charger 804, AFE and controller in biosignal signal monitor 802 can detect whether biosignal sensors (e.g., electrodes) of biosignal monitor 802 are attached to a user or not.

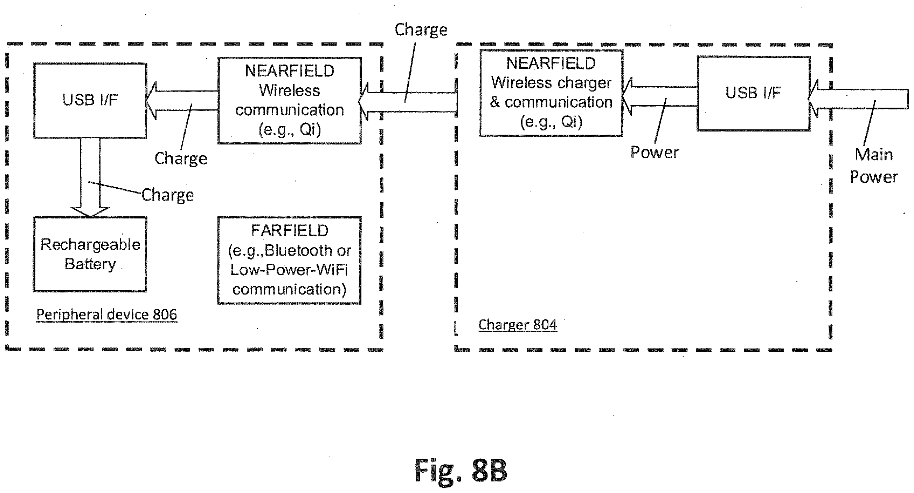

Fig. 8B is a diagram showing wireless battery charging using the nearfield wireless interface when implementing a biosignal monitoring system with detachable electronics and wireless rechargeable battery in a peripheral device in one embodiment. A peripheral device 806, such as an insulin pump, can be used concurrently with biosignal monitors as shown in Fig. 5C, Fig. 6A and Fig. 6B. Peripheral device 806 can include a rechargeable battery. In one embodiment, the rechargeable battery in peripheral device 806 can be charged using a higher power nearfield protocol (when compared to NFC), such as Qi protocol. In an aspect, the higher power nearfield charging can provide power to rechargeable batteries in peripheral device 806 that may be larger and may require more power when compared to the biosignal monitor 802, whose rechargeable battery can be much smaller. Charger 804 can receive main power from a power source, such as an external battery or a power supply. When charger 804 is brought into a specific distance, such as distance that complies with the Qi protocol, charger 804 can provide power to charge rechargeable battery in peripheral device 806.

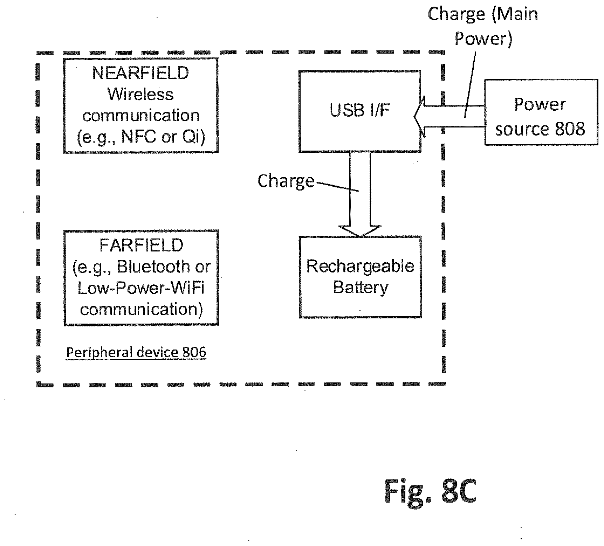

Fig. 8C is a diagram showing wired battery charging when implementing a biosignal monitoring system with detachable electronics and wireless rechargeable battery in a peripheral device in one embodiment. In one embodiment, in addition to wireless charging, rechargeable battery in peripheral device 806 can also be charged using wired connection. Example wired connections that can be used for the wired charging can include, for example, various types of universal serial bus (USB) protocols. In embodiments where wired charging is implemented, peripheral device 806 can include power interfaces such as a USB interface or socket. A power source 808, which can be battery or another device such as smartphone, laptop, personal computer, or the like, can provide main power directly to the rechargeable battery in biosignal monitor 802 using a wired connection. In one embodiment, the USB interface (I/F) shown in Fig. 8C can operate under USB Type C Power Delivery (USB-C-PD) Protocol to fast charge the rechargeable battery in the peripheral device 806 (e.g., insulin pump) in order to keep the charging time short, in situations where the user may not be able to move away from the power source 808. To ensure personal health and safety, the USB connection in one embodiment may be physically made by using magnets and spring-loaded (e.g., gold) contacts. If the user moves further than the charging cable would allow, it is then automatically detached without hurting the user.

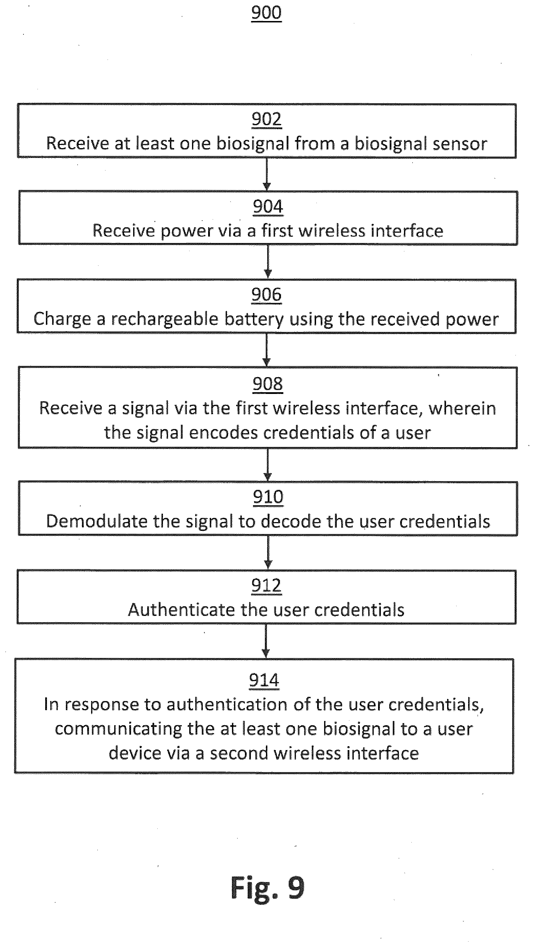

Fig. 9 illustrates a flow diagram of a process to implement a flow diagram of a process to implement a biosignal monitoring system with detachable electronics and wireless rechargeable battery in one embodiment. The process 900 shown in Fig. 9 can include one or more operations, actions, or functions as illustrated by one or more of blocks 902, 904, 906, 908, 910, 912 and/or 914 Although illustrated as discrete blocks, various blocks can be divided into additional blocks, combined into fewer blocks, eliminated, performed in different order, or performed in parallel, depending on the desired implementation.

Process 900 can be performed by a device that can be reusable (e.g., reusable portion shown in Fig. 2) connected to a disposable biosignal sensor. Process 900 can begin at block 902. At block 902, the device can receive and/or evaluate at least one biosignal from a biosignal sensor (e.g., disposable biosignal sensor). In one embodiment, the at least one biosignal can include glucose values. Process 900 can proceed from block 902 to block 904. At block 904, the device can receive power via a first wireless interface. Process 900 can proceed from block 904 to block 906. At block 906, the device can charge a rechargeable battery using the received power. Process 900 can proceed from block 906 to block 908. At block 908, the device can receive a signal via the first wireless interface, wherein the signal encodes credentials of a user. Process 900 can proceed from block 908 to block 910. At block 910, the device can demodulate the signal to decode the user credentials.

Process 900 can proceed from block 910 to block 912. At block 912, the device can authenticate the user credentials. Process 900 can proceed from block 912 to block 914. At block 914, the device can, in response to authentication of the user credentials, communicating the at least one biosignal to a user device via a second wireless interface. In one embodiment, the first wireless interface can be a nearfield wireless interface and the second wireless interface can be a farfield wireless interface. In one embodiment, the first wireless interface can be one of a near field communication (NFC) interface and Qi wireless power transfer interface. The second wireless interface can be one of a Bluetooth interface or a WiFi interface or an LTE interface (e.g., low power LTE-M or NB-IoT).

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The corresponding structures, materials, acts, and equivalents of all means or step plus function elements, if any, in the claims below are intended to include any structure, material, or act for performing the function in combination with other claimed elements as specifically claimed. The description of the present invention has been presented for purposes of illustration and description, but is not intended to be exhaustive or limited to the invention in the form disclosed. Many modifications and variations will be apparent to those of ordinary skill in the art without departing from the scope and spirit of the invention. The embodiment was chosen and described in order to best explain the principles of the invention and the practical application, and to enable others of ordinary skill in the art to understand the invention for various embodiments with various modifications as are suited to the particular use contemplated.

## Claims

1. A device comprising:
an analog front end configured to receive at least one biosignal from one or more biosignal sensors;
a wireless communication receiver comprising a first wireless interface, wherein the wireless communication receiver is configured to:
receive power via the first wireless interface;
charge a rechargeable battery using the received power; and
receive a signal via the first wireless interface, wherein the signal encodes credentials of a user;
a second wireless interface; and
one or more controllers configured to:
demodulate the signal to decode the user credentials;
authenticate the user credentials; and
in response to authentication of the user credentials, communicate the at least one biosignal from the analog front end to a user device via the second wireless interface.

2. The device of claim 1, wherein the first wireless interface is a nearfield wireless interface and the second wireless interface is a farfield wireless interface.

3. The device of claim 1, wherein:
the first wireless interface is one of a near field communication (NFC) interface and Qi wireless power transfer interface; and
the second wireless interface is one of a Bluetooth interface, a WiFi interface and a Long-Term Evolution (LTE) interface.

4. The device of claim 1, wherein the analog front end is detachable from the biosignal sensor.

5. The device of claim 1, wherein the analog front end is attached to the biosignal sensor via at least one mechanical fixation.

6. The device of claim 1, wherein the at least one biosignal comprises glucose values.

7. The device of claim 1, wherein the signal further encodes at least one of encryption keys and authentication information.

8. The device of claim 1, wherein the one or more controllers are configured to:
in response to authentication of the user credentials, encrypt the at least one biosignal; and
communicate the encrypted biosignals to the user device via the second wireless interface.

9. A system comprising:
a first device configured to sense at least one biosignal; and
a second device configured to:
receive the at least one biosignal from the first device;
receive power via a first wireless interface;
charge a rechargeable battery using the received power;
receive a signal via the first wireless interface, wherein the signal encodes credentials of a user;
demodulate the signal to decode the user credentials;
authenticate the user credentials; and
in response to authentication of the user credentials, communicate the at least one biosignal to a user device via a second wireless interface.

10. The system of claim 9, wherein the first wireless interface is a nearfield wireless interface and the second wireless interface is a farfield wireless interface.

11. The system of claim 9, wherein:
the first wireless interface is one of a near field communication (NFC) interface and Qi wireless power transfer interface; and
the second wireless interface is one of a Bluetooth interface, a WiFi interface and an Long-Term Evolution (LTE) interface.

12. The system of claim 9, wherein the second device is detachable from the first device.

13. The system of claim 9, wherein the second device is attached to the first device via at least one mechanical fixation.

14. The system of claim 9, wherein the at least one biosignal comprises glucose values.

15. The system of claim 9, wherein the signal further encodes at least one of encryption keys and authentication information.

16. The system of claim 9, wherein the second device is further configured to:
in response to authentication of the user credentials, encrypt the at least one biosignal; and
communicate the encrypted biosignals to the user device via the second wireless interface.

17. A method for operating a biosignal monitoring system, the method comprising:
receiving at least one biosignal from a biosignal sensor;
receiving power via a first wireless interface;
charging a rechargeable battery using the received power;
receiving a signal via the first wireless interface, wherein the signal encodes credentials of a user;
demodulating the signal to decode the user credentials;
authenticating the user credentials; and
in response to authentication of the user credentials, communicating the at least one biosignal to a user device via a second wireless interface.

18. The method of claim 17, wherein the first wireless interface is a nearfield wireless interface and the second wireless interface is a farfield wireless interface.

19. The method of claim 17, wherein:
the first wireless interface is one of a near field communication (NFC) interface and Qi wireless power transfer interface; and
the second wireless interface is one of a Bluetooth interface, a WiFi interface and a Long-Term Evolution (LTE) interface.

20. The method of claim 17, wherein the at least one biosignal comprises glucose values.
